# EUROPÄISCHE PATENTSCHRIFT

(11) **EP 0 518 875 B1**
(45) Veröffentlichungstag und Bekanntmachung des Hinweises auf die Patenterteilung: **21.09.1994**
(21) Anmeldenummer: 91903794.5
(22) Anmeldetag: 21.02.1991
(51) Int. Cl.: C07C 303/08, C07C 303/12, C07C 309/86

(54) **VERFAHREN ZUR HERSTELLUNG VON 3-NITROBENZOLSULFONSÄURECHLORID**
METHOD FOR PREPARING 3-NITROBENZENE SULPHONIC ACID CHLORIDE
PROCEDE DE PRODUCTION DE CHLORURE D'ACIDE 3-NITROBENZENESULFONIQUE

(30) Priorität: 03.03.1990 DE 4006666
(43) Veröffentlichungstag der Anmeldung: 23.12.1992
(73) Patentinhaber: HOECHST AKTIENGESELLSCHAFT, 65926 Frankfurt am Main (DE)
(72) Erfinder: MEIER, Michael, Corpus Christi, TX 78412 (US); WAGNER, Reinhard, D-6200 Wiesbaden (DE)
(86) Internationale Anmeldenummer: EP9100322
(87) Internationale Veröffentlichungsnummer: WO9113863

(56) Entgegenhaltungen:
- EP-A- 0 289 847
- EP-A- 0 403 947
- DE-C- 89 997
- FR-A- 2 360 568
- US-A- 3 574 739
- CHEMICAL ABSTRACTS, vol. 101, 1984, Columbus, Ohio, US; see p. 613, abstract 210737a, (& SU-A-1097613), 15 June 1984
- CHEMICAL ABSTRACTS, vol. 113, No. 9, 27 August 1990, Columbus, Ohio, US; see abstract 77909n, & JP-A-02108661

## Beschreibung

Die Erfindung betrifft ein verbessertes Verfahren zur Herstellung von 3-Nitrobenzolsulfonsäurechlorid in hohen Ausbeuten durch Umsetzung von Nitrobenzol mit Chlorsulfonsäure und anschließende Einwirkung eines anorganischen Säurechlorids.

3-Nitrobenzolsulfonsäurechlorid stellt ein wichtiges Vorprodukt dar, insbesondere zur Herstellung von Farbstoffen.

Es ist bekannt (BIOS Final Report No. 1153, S. 38), 3-Nitrobenzolsulfonsäurechlorid durch Sulfochlorierung von Nitrobenzol bei Temperaturen von 60°C - 105°C herstellen zu können. Bei diesem Verfahren wird das Nitrobenzol zu 4 mol Chlorsulfonsäure gegeben, wobei die Temperatur langsam auf 60°C ansteigt. Nach beendeter Zugabe wird die Reaktionsmischung langsam auf 70°C und dann bis auf 105°C erhitzt und schließlich mehrere Stunden bei 105°C gehalten. Die Ausbeute bei diesem Verfahren wurde mit 79 % d.Th. angegeben, was für ein großtechnisches Verfahren nicht ausreicht. Es ist ferner bekannt (C.M. Suter, "The Organic Chemistry of Sulfur", John Wiley & Sons Inc., New York/London, 1944, Seite 223), daß beim Erhitzen von Nitrobenzol mit 2 Moläquivalenten Chlorsulfonsäure angeblich 3-Nitrobenzolsulfonsäurechlorid entsteht (was in neuerer Literatur wieder in Frage gestellt wurde), wobei beim Erhitzen auf 150°C über mehrere Stunden heftige Zersetzung des Reaktionsgemisches unter Bildung von SO₂ auftritt.

Die Herstellung von 3-Nitrobenzolsulfonsäurechlorid durch Chlorierung von 3-Nitrobenzolsulfonsäure mit Chlor/Dischwefeldichlorid ist gleichfalls beschrieben (Chem. Abstr. 104 [1986] 68 593s). 3-Nitrobenzolsulfonsäurechlorid konnte auch durch Umsetzung von 3-Nitrobenzolsulfonsäure-Na-Salz mit Phosphoroxychlorid in Sulfolan hergestellt werden (Chem. Abstr. 98 [1983] 215 331 t).

Schließlich konnte das gewünschte 3-Nitrobenzolsulfonsäurechlorid auch aus der Umsetzung von Benzol mit Chlorsulfonsäure und einem Gemisch aus konz. Salpetersäure und konz. Schwefelsäure in einer Ausbeute von ca. 90 % (SU 1097613 [1982]) erhalten werden.

Es bestand daher ein Bedürfnis nach einem ökonomisch und ökologisch vorteilhaften Verfahren zur Herstellung von 3-Nitrobenzolsulfonsäurechlorid in hohen Ausbeuten.

Es wurde nun überraschenderweise gefunden, daß man 3-Nitrobenzolsulfonsäurechlorid in vorteilhafter Weise in hohen Ausbeuten aus Nitrobenzol und Chlorsulfonsäure herstellen kann, indem man Nitrobenzol mit Chlorsulfonsäure bei Temperaturen von etwa 90 bis etwa 120°C umsetzt und auf das erhaltene Reaktionsgemisch ein anorganisches Säurechlorid bei etwa 40 bis etwa 90°C, vorzugsweise bei etwa 60 bis etwa 80°C, einwirken läßt.

Das Verfahren wird im einzelnen zweckmäßigerweise so durchgeführt, daß man die Chlorsulfonsäure bei einer Temperatur von etwa 90 bis etwa 120°C, bevorzugt von etwa 110 bis etwa 115°C, vorlegt und das Nitrobenzol zudosiert. Wenn die Gesamtmenge an Nitrobenzol zugegeben ist, hält man die Reaktionsmischung vorteilhaft noch etwa 4 Stunden in den angegebenen Temperaturbereichen und läßt sie danach auf etwa 40 bis etwa 90°C abkühlen. In diesem Temperaturbereich, bevorzugt jedoch bei etwa 60 bis etwa 80°C, wird anschließend das anorganische Säurechlorid zudosiert. Es ist vorteilhaft, die Reaktionsmischung dann noch etwa 2 bis 3 Stunden in diesem Temperaturbereich zu halten.

Als anorganische Säurechloride können beispielsweise Phosphoroxychlorid, Phosphortrichlorid oder Thionylchlorid eingesetzt werden. Phosgen ist ebenfalls verwendbar und soll von dem Oberbegriff "anorganisches Säurechlorid" mit umfaßt werden. Besonders bevorzugt ist die Verwendung von Thionylchlorid.

Beim erfindungsgemäßen Verfahren werden pro mol Nitrobenzol etwa 3,0 bis etwa 10,0 mol, vorzugsweise etwa 3,5 bis etwa 6,0 mol, besonders bevorzugt etwa 4,0 bis etwa 4,6 mol Chlorsulfonsäure eingesetzt. Verwendet man weniger als 3 mol Chlorsulfonsäure pro mol Nitrobenzol, so kommt man in Bereiche, in denen die Reaktionslösung leichter zur Zersetzung neigt. Mehr als 10 mol Chlorsulfonsäure einzusetzen ist zwar möglich, aber wenig sinnvoll, da hierbei ohne merklichen Vorteil nur der Überschuss an später zu entsorgender Chlorsulfonsäure zunimmt.

Das erfindungsgemäße Verfahren schließt die kontinuierliche Fahrweise ein. Sie wird dadurch erreicht, daß man z.B. in einer Kaskade aus mindestens zwei Kesseln in den ersten Kessel bei konstanter Temperatur von z.B. 110-115°C Chlorsulfonsäure und Nitrobenzol einlaufen läßt. Mit Hilfe eines Überlaufes stellt man eine gewünschte mittlere Verweilzeit ein. In einem zweiten Kessel hält man eine Temperatur von beispielsweise 60-80°C und dosiert die gewünschte Menge anorganisches Säurechlorid, beispielsweise Thionylchlorid, zu. Es ist möglich, jedoch nicht zwingend, die Reaktionslösung vor ihrer Aufarbeitung in einem dritten Kessel nachreagieren zu lassen.

Die Kaskade kann durch eine andere kontinuierlich arbeitende Apparatur, beispielsweise einen Umpumpreaktor oder einen Röhrenreaktor ersetzt werden.

Das anorganische Säurechlorid wird in einer Menge von etwa 0,1 bis etwa 5,0 mol, vorzugsweise von etwa 0,2 bis etwa 1,0 mol, pro mol Nitrobenzol verwendet.

Das erfindungsgemäße Verfahren kann sowohl bei Normaldruck als auch bei Überdruck durchgeführt werden.

Mit Hilfe des erfindungsgemäßen Verfahrens erhält man das gewünschte 3-Nitrobenzolsulfonsäurechlorid in einer Ausbeute von etwa 95 bis etwa 98 % d.Th. Die hohe Ausbeute bringt als weiteren Vorteil mit sich, daß das Abwasser praktisch nicht mehr mit Nitrobenzol oder Nitrobenzolsulfonsäure belastet ist.

Die erfindungsgemäß erhaltenen Reaktionsgemische weisen überraschenderweise hohe thermische Stabilität auf, wodurch die Sicherheit der Reaktionsführung erhöht ist. Die Differentialthermoanalyse zeigt, daß exotherme Zersetzung erst oberhalb von 180°C einsetzt.

3-Nitrobenzolsulfonsäurechlorid kann durch Eintragen des Reaktionsgemisches in Eiswasser und Absaugen des ausgefallenen Produktes isoliert werden. Das erhaltene Rohprodukt ist von hoher Reinheit und kann ohne weitere Reinigung eingesetzt werden.

Durch das nachstehende Beispiel wird das erfindungsgemäße Verfahren erläutert, ohne darauf beschränkt zu werden.

### Beispiel

Zu 521,0 g (4,4 mol) Chlorsulfonsäure wurden bei 112°C 123,1 g (1,0 mol) Nitrobenzol über 4 Stunden zugetropft. Anschließend wurde bei dieser Temperatur 4 Stunden nachgerührt. Nachdem auf 70°C abgekühlt worden war, wurden 110,0 g (0,92 mol) Thionylchlorid über 2 Stunden zugetropft.

Dann wurde bei dieser Temperatur bis zum Ende der Gasentwicklung nachgerührt. Anschließend wurde die Reaktionsmischung abgekühlt und bei 5°C in Eiswasser eingetragen. Anschließend wurde das ausgefallene 3-Nitrobenzolsulfonsäurechlorid abgesaugt und mit Wasser und mit Natriumhydrogencarbonat gewaschen. Auf diese Weise wurden 237,5 g feuchtes 3-Nitrobenzolsulfonsäurechlorid mit einem Reingehalt von 89,9 % erhalten. Der Trockengehalt betrug 213,5 g, was einer Ausbeute von 96,3 % entspricht.

## Patentansprüche

1. Verfahren zur Herstellung von 3-Nitrobenzolsulfonsäurechlorid aus Nitrobenzol und Chlorsulfonsäure, dadurch gekennzeichnet, daß man Nitrobenzol mit Chlorsulfonsäure bei etwa 90 bis etwa 120°C umsetzt und auf das erhaltene Reaktionsgemisch anschließend ein anorganisches Säurechlorid bei etwa 40 bis etwa 90°C einwirken läßt.

2. Verfahren nach Anspruch 1, dadurch gekennzeichnet, daß man die Umsetzung von Nitrobenzol mit Chlorsulfonsäure bei etwa 110 bis etwa 115°C durchführt.

3. Verfahren nach mindestens einem der Ansprüche 1 und 2, dadurch gekennzeichnet, daß man die Nachbehandlung mit dem anorganischen Säurechlorid bei einer Temperatur von etwa 60 bis etwa 80°C durchführt.

4. Verfahren nach mindestens einem der Ansprüche 1 bis 3, dadurch gekennzeichnet, daß als anorganisches Säurechlorid Thionylchlorid verwendet wird.

5. Verfahren nach mindestens einem der Ansprüche 1 bis 4, dadurch gekennzeichnet, daß man pro mol Nitrobenzol etwa 3,0 bis etwa 10,0 mol Chlorsulfonsäure einsetzt.

6. Verfahren nach mindestens einem der Ansprüche 1 bis 5, dadurch gekennzeichnet, daß man pro mol Nitrobenzol etwa 3,5 bis etwa 6,0 mol Chlorsulfonsäure einsetzt.

7. Verfahren nach mindestens einem der Ansprüche 1 bis 6, dadurch gekennzeichnet, daß man pro mol Nitrobenzol etwa 4,0 bis etwa 4,6 mol Chlorsulfonsäure einsetzt.

8. Verfahren nach mindestens einem der Ansprüche 1 bis 7, dadurch gekennzeichnet, daß man pro mol Nitrobenzol etwa 0,1 bis etwa 5,0 mol anorganisches Säurechlorid einsetzt.

9. Verfahren nach mindestens einem der Ansprüche 1 bis 8, dadurch gekennzeichnet, daß man pro mol Nitrobenzol etwa 0,2 bis etwa 1,0 mol anorganisches Säurechlorid einsetzt.

10. Verfahren nach mindestens einem der Ansprüche 1 bis 9, dadurch gekennzeichnet, daß man das Verfahren diskontinuierlich oder kontinuierlich durchführt.

11. Verfahren nach mindestens einem der Ansprüche 1 bis 10, dadurch gekennzeichnet, daß man das Verfahren bei Normaldruck oder Überdruck durchführt.

## Claims

1. A process for the preparation of 3-nitrobenzenesulfonyl chloride from nitrobenzene and chlorosulfonic acid, which comprises reacting nitrobenzene with chlorosulfonic acid at about 90 to about 120°C and allowing an inorganic acid chloride to act on the resulting reaction mixture at about 40 to about 90°C.

2. The process as claimed in claim 1, wherein the reaction of nitrobenzene with chlorosulfonic acid is carried out at about 110 to about 115°C.

3. The process as claimed in at least one of claims 1 and 2, wherein the subsequent treatment with the inorganic acid chloride is carried out at a temperature of about 60 to about 80°C.

4. The process as claimed in at least one of claims 1 to 3, wherein thionyl chloride is used as the inorganic acid chloride.

5. The process as claimed in at least one of claims 1 to 4, wherein about 3.0 to about 10.0 mol of chlorosulfonic acid are employed per mol of nitrobenzene.

6. The process as claimed in at least one of claims 1 to 5, wherein about 3.5 to about 6.0 mol of chlorosulfonic acid are employed per mol of nitrobenzene.

7. The process as claimed in at least one of claims 1 to 6, wherein about 4.0 to about 4.6 mol of chlorosulfonic acid are employed per mol of nitrobenzene.

8. The process as claimed in at least one of claims 1 to 7, wherein about 0.1 to about 5.0 mol of inorganic acid chloride are employed per mol of nitrobenzene.

9. The process as claimed in at least one of claims 1 to 8, wherein about 0.2 to about 1.0 mol of inorganic acid chloride is employed per mol of nitrobenzene.

10. The process as claimed in at least one of claims 1 to 9, wherein the process is carried out batchwise or continuously.

11. The process as claimed in at least one of claims 1 to 10, wherein the process is carried out at normal pressure or elevated pressure.

## Revendications

1. Procédé de préparation du chlorure de 3-nitrobenzène-sulfonyle à partir de nitrobenzène et d'acide chlorosulfonique, procédé caractérisé en ce que l'on fait réagir le nitrobenzène avec l'acide chlorosulfonique à des températures d'environ 90 à 120°C puis on fait agir sur le mélange de réaction obtenu un chlorure d'acide minéral à des températures de l'ordre de 40 à 90°C.

2. Procédé selon la revendication 1, caractérisé en ce que l'on effectue la réaction du nitrobenzène avec l'acide chlorosulfonique à des températures d'environ 110 à 115°C.

3. Procédé selon la revendication 1 ou 2, caractérisé en ce que l'on effectue le traitement avec le chlorure d'acide minéral à une température d'environ 60 à 80°C.

4. Procédé selon une ou plusieurs des revendications 1 à 3, caractérisé en ce que l'on utilise le chlorure de thionyle comme chlorure d'acide minéral.

5. Procédé selon une ou plusieurs des revendications 1 à 4, caractérisé en ce que l'on fait réagir environ 3,0 à 10,0 mol d'acide chlorosulfonique par moi de nitrobenzène.

6. Procédé selon une ou plusieurs des revendications 1 à 5, caractérisé en ce que l'on fait réagir environ 3,5 à 6,0 mol d'acide chlorosulfonique par mol de nitrobenzène.

7. Procédé selon une ou plusieurs des revendications 1 à 6, caractérisé en ce que l'on fait réagir environ 4,0 à 4,6 mol d'acide chlorosulfonique par mol de nitrobenzène.

8. Procédé selon une ou plusieurs des revendications 1 à 7, caractérisé en ce que l'on fait réagir environ 0,1 à 5,0 mol du chlorure d'acide minéral par mol de nitrobenzène.

9. Procédé selon une ou plusieurs des revendications 1 à 8, caractérisé en ce que l'on fait réagir environ 0,2 à 1,0 mol du chlorure d'acide minéral par mol de nitrobenzène.

10. Procédé selon une ou plusieurs des revendications 1 à 9, caractérisé en ce qu'on l'effectue en discontinu ou en continu.

11. Procédé selon une ou plusieurs des revendications 1 à 10, caractérisé en ce qu'on l'effectue à la pression normale ou sous pression.
